# EUROPEAN PATENT APPLICATION

(11) **EP 0 527 281 A1**
(43) Date of publication of application: **17.02.1993**
(21) Application number: 91307517.2
(22) Date of filing: 14.08.1991
(51) Int. Cl.: A61F 15/00

(54) **Cottonbud dispenser**

(71) Applicant: Wu, Ching-Kao, Taipei (TW)
(72) Inventor: Wu, Ching-Kao, Taipei (TW)
(74) Representative: Cardwell, Stuart Martin

(57) **Abstract**

A cottonbud dispenser consisting of a base 2, a plurality of long coiled springs 3, pushing blocks 4 to push said springs, a position block 5, a fixing plate 6, a stop plate 7 and a transparent case body 8 storing cottonbuds, any of said pushing blocks below the base being possible to be pushed to the left to push a cottonbud in one of the rooms in the base to extend out of the said room so as to be picked up by fingers and also possible to be pushed back to its original position by the compressed long spring after said pushing block is released.

## Description

The present invention relates to a cottonbud dispenser.

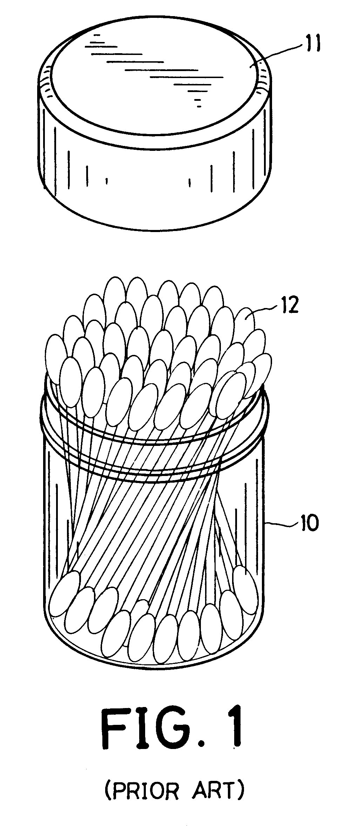

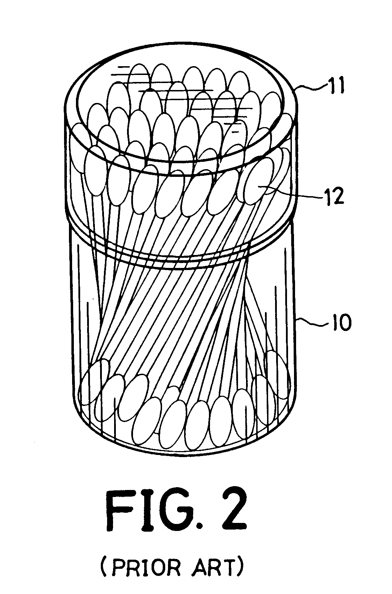

A conventional cottonbud or swab case as shown in Figures 1 and 2 generally has a cylindrical container 10 closed with a lid 11, which may often be taken off with excessive force by a user as to force some of the cottonbuds stored therein to drop out of the container 10. In addition, extra cottonbuds may be carelessly and easily be pulled out together with the one chosen to be picked up, and if worse, the cotton balls at both ends of sticks may be contaminated in picking up. Spreading out upper ends, as shown in Figure 1, of cottonbuds should be pushed to stand upright before the lid 11 is to be covered on the container 10, which it quite inconvenient to handle in covering the lid 11 every time it is taken off in picking up a cottonbud.

In view of the disadvantage of a conventional cottonbud case, this cottonbud dispenser has been devised to have the following advantages.
1. Cottonbuds are stored lengthwise in a case body and can be pushed out one by one by a pushing block manually pulled so as to be picked up by fingers without touching cotton balls at both ends of a stick.
2. A case body stored with cottonbuds can be easily replaced by a new case body fully stored with cottonbuds if all of the cottonbuds in old one are used up.
3. Cottonbuds stored in the case body can be taken out one by one orderly without inconvenience of dropping out of the case body.

The present invention will now be described further, by way of example only, with reference to the accompanying drawings, in which:-
Figure 1 is a perspective view of a conventional cottonbud case with cottonbuds stored in,
Figure 2 is a perspective view of a conventional cottonbud case,
Figure 3 is an exploded perspective view of a cottonbud dispenser in accordance with the present invention,
Figure 4 is a perspective view of a cottonbud dispenser in accordance with the present invention,
Figure 5 is a cross-sectional view of a cottonbud dispenser in accordance with the present invention,
Figure 6 is a cross-sectional view of a cottonbud dispenser with a cottonbud dealt out in accordance with the present invention, and
Figure 7 is a perspective view of the case body to be taken off the base with the fixing plate pulled down in accordance with the present invention.

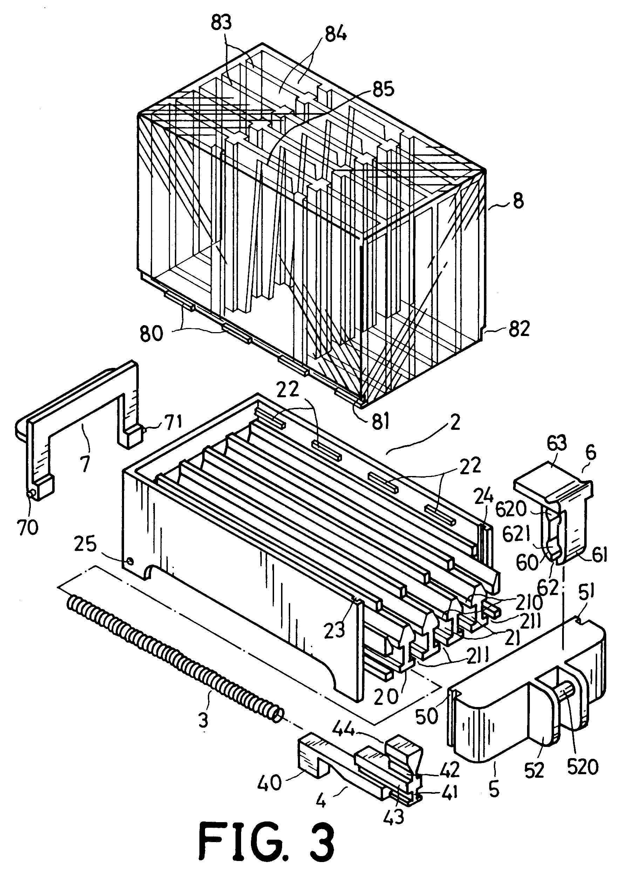

Reference is now made to Figures 3 to 7.

A cottonbud dispenser in accordance with the present invention, as shown in Figure 3 comprises a rectangular base 2, a plurality of long coiled springs 3, the same number of pushing blocks 4 as the springs 3, a position block 5, a reverse L-shaped fixing plate 6, a reverse U-shaped stop plate 7 and a case body 8 as the main components.

The rectangular base 2 has its interior divided with a plurality of lengthwise separating boards 20 equally spaced apart to form a plurality of rooms 21, each of which has a lengthwise opening 210, 211 respectively at the upper and the bottom. The base 2 also has a row of several short ridges 22 spaced apart on both lenghtwise upper inner walls, vertical grooves 23, 24 respectively in both right ends of the lengthwise inner walls, a fitting hole 25, 26(not shown in the drawings) in both right ends of the lengthwise sides, the same number of exits 27 as the rooms 21 in the left side communicating with the rooms 21.

The same number of long coiled springs 3 and of pushing blocks 4 as the rooms 21 are respectively placed in each room 21 in series so that said blocks 4 can manually push said springs 3.

The pushing blocks 4 are provided with a pulling grip 40 extending down at the left side, two I-shaped guide rails 41, 42 at the right upper section sandwiching a blocker 43, and a pushing piece 44 on the rail 42.

The position block 5 is to be combined with the base 2, having two vertical ridges 50, 51 respectively on both sides of the left vertical wall to fit in the vertical guide grooves 23, 24 of the base 2, a rod supporter 52 and a rod 520 held between two parallel walls of the rod supporter 52.

The reverse L-shaped fixing plate 6 is to be sustained on the rod 520 in the supporter 52, having two downward spaced-apart pinch arms 60, 61 forming an opening 62 and two circular holes 620, 621 in line and a flat square block 63 extending leftward at the top.

The reverse U-shaped stop plate 7 is to be combined with the base 2 at its left side to normally stop the exits 27 in the left side of the base 2, having two projections 70, 71 respectively at the lower ends of the two vertical portions to fit in fitting holes 25, 26 in the base 2 to male said stop plate 7 turn down with the projections 70, 71 as pivots.

The rectangular case body 8 is made of transparent material to be positioned on the base 2, having four side walls and a top wall but no bottom wall, several spaced-apart ridges 80 in line on both bottom edges of the lengthwise sides to form two guide grooves 81, 82, a plurality of same-sized separating boards 83 equally spaced apart in the interior, and two neighghboring boards 83 forming a room 84 for storing cottonbuds horizontally and lengthwise. Each separating board 83 has a large opening in the middle portion facing to the bottom. The number of the rooms 84 is the same as that of the rooms 21 in the base 2. To load cottonbuds in the case body 8, said case body 8 is to be held upside down, and cottonbuds are to be put in each room 84 through the bottom. When the case body has been loaded fully with cottonbuds, then said case body can be assembled with the base 2 also held upside down on the case body 8, which is to be pulled from the right to the left making the ridges 80 to fit in the spaces among the ridges 22 so as to male the case body 8 sit on the base 2 and each room 84 lengthwise communicate with each room 21 in the base so that the lowest cottonbud in each room 84 can go down in the corresponding room 21 in the base 2.

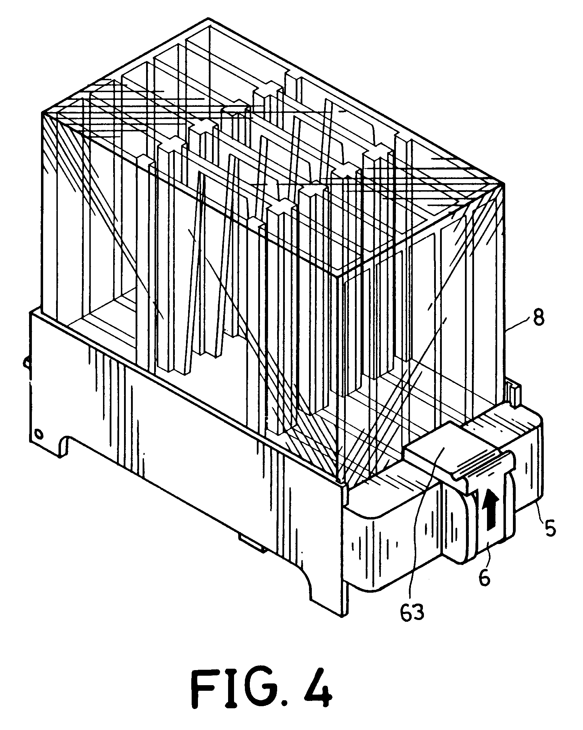

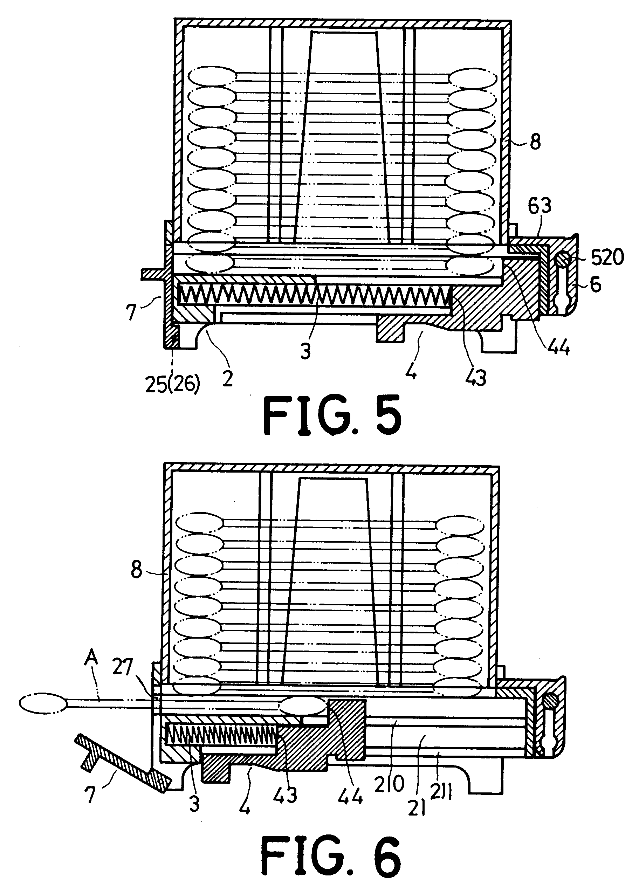

In assembling this cottonbud dispenser, at first, the springs 3 and the pushing blocks 4 are to be placed in series in the rooms 21 in the base 2, with one end of each spring 3. resting on the left end of the blocker 43 of each pushing block 4. At the same time, the pulling grip 40 extends downward out of each room 21, the guide rails 41, 42 respectively fit and can move in the lengthwise openings 210, 211, and the pushing piece 44 extends in the opening 210. Next, the position block 5 is to be combined with the base 2, by fitting both ridges 50, 51 in the guide grooves 23, 24. Then the case body 8 stored with cottonbuds is to be assembled with the base 2, by holding said case body 8 upside down and the base 2 also upside down and pushing said case body 8 from the right to the left under the base 2, with the ridges 80 fitting in the spaces among the ridges 22. Then they are to be turned upside down again after assembled together. Next, the reverse L-shaped fixing plate 6 is to be combined with the position block 5, by pushing down said palte 6 and placing the circular hole 620 fitting with the rod 52 as shown in Figs. 4 and 5. Thus, the flat block 63 of the reverse L-shaped fixing plate 6 can keep the case body 8 in place, preventing the case body 8 from moving off the base 2. Lastly, the reverse U-shaped stop plate 7 is to be combined with the base 2, with the projections 70, 71 fitting in the holes 25, 26 and making the stop plate 6 to stand close beside the left side of the base 2 so as to keep the cottonbuds in the case body from moving out of the exits 27.

In order to take out a cottonbud from the cottonbud dispenser, any of the pushing blocks 4 below the base 2 as shown in Fig. 6 can be pushed with a finger to the left, and the pushing piece 44 will push a cottonbud A to the left out of one of the rooms 21 to be picked by fingers. And simultaneously, the blocker 43 will force and compress one of the springs 3, and the reverse U-shaped stop plate 7 will be pushed open by the cottonbud A. After the cottonbud has been taken out of the base 2, the pushing block 4 can elastically be pushed back to its original position by the compressed spring 3 elastically lengthening as shown in Fig. 5, and becomes ready for next manipulation, after the lowest cottonbud in the room 84 has dropped down in the room 21 where the cottonbud had just been taken out from.

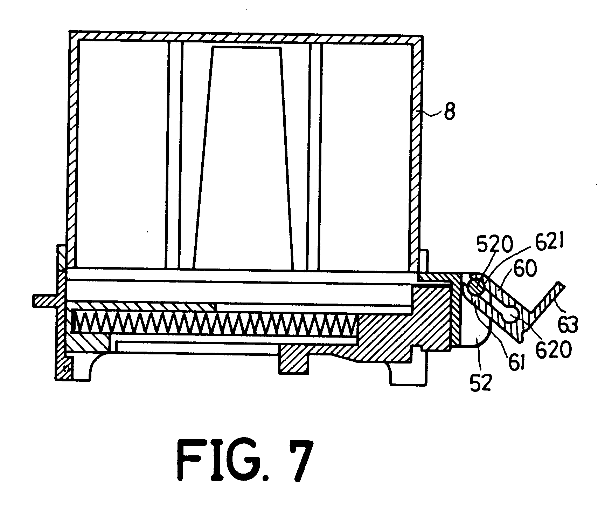

When all of the cottonbuds A stored in the case body 8 have been taken out, the reverse L-shaped filing plate 6 is to be pulled up as shown in Fig. 7, placing the lower circular hole 621 to fit with the rod 520, and then said plate 6 is to be pulled down to the right with the rod 520 as a pivot. Now the flat plate 63 can no longer keep the case body 8 in its position, which can be taken off the base 2, by pulling the case body 8 horizontally from the left to the right. Then a new case body 8 loaded with cottonbuds can be assembled with the base 2 in place of the old one, in the way mentioned above.

## Claims

1. A cottonbud dispenser comprising a base and a case body storing cottonbuds and presenting same to at least one dispensing room, characterised by a respective pushing block for each dispensing room which pushing block is received slidably in the base and is operable to push a cottonbud in said room to extend out of the room so as to be picked up by fingers.

2. A cottonbud dispenser as claimed in claim 1 further comprising a respective spring for each pushing block and operative to return the pushing block to its original position after said pushing block is released.

3. A cottonbud dispenser as claimed in claim 1 or 2 in which the base has its interior divided into a plurality of lengthwise rooms separated equally with separating vertical boards, each of said rooms being provided with a lengthwise opening at the upper and the bottom portion and the same number of exits at the left side as that of the rooms.

4. A cottonbud dispenser as claimed in claim 3 wherein said base also has a row of spaced apart several ridges on both inner upper lengthwise walls, a vertical guide groove respectively in the inner right ends of both lengthwise sides and a fitting hole respectively at the left lower end portions of both lengthwise sides.

5. A cottonbud dispenser as claimed in any one of claim 1 to 4 in which there are as many springs as the rooms in the base, and the springs are respectively positioned in each room to be pushed and compressed by a blocker in a pushing block.

6. A cottonbud dispenser as claimed in any one of the preceding claims in which there are as many pushing blocks as the rooms in the base, and which are combined with the base partly extending down from said rooms, having a pulling grip extending downward, two guide rails sandwiching a blocker extending upward from the pulling grip and having its left end rested on by one end of the spring and a pushing piece on the upper guide rails.

7. A cottonbud dispenser as claimed in claim 4 further comprising a position block to be combined with the base, having two opposite vertical ridges to fit in the two vertical guide grooves in the base, and a rod supporter consisting of two parallel vertical walls and a rod pinched between said walls.

8. A cottonbud dispenser as claimed in claim 7 comprising a fixing plate to be sustained on the rod in the position block, having two spaced apart downward pinch arms forming an upper and a lower circular hole and an opening, and a flat square plate extending to the left from the top, said upper and lower circular hole being selectably fitted in by the rod in the position block.

9. A cottonbud dispenser as claimed in any one of the claims 4 to 8 comprising a stop plate to be combined with the base at its left side, having two opposite projections at both lower sides to fit in the two fitting holes in the base, said stop plate normally closing a plurality of exits in the left side of the base and to be pushed open by a cottonbud pushed by the pushing block out of one of the rooms in the base.

10. A cottonbud dispenser as claimed in any one of the preceding claims in which the case body to be assembled on the base, has its interior divided into a plurality of lengthwise rooms with separating vertical boards equally spaced apart for storing cottonbuds lengthwise, each of said separating vertical boards having a large opening at the middle portion facing to the bottom.
